# EUROPEAN PATENT SPECIFICATION

(11) **EP 1 843 838 B1**
(45) Date of publication and mention of the grant of the patent: **08.06.2011**
(21) Application number: 05854902.3
(22) Date of filing: 19.12.2005
(51) Int. Cl.: B01J 19/12

(54) **PHOTOCHLORINATION AND DEHYDROHALOGENATION PROCESS FOR PREPARATION OF OLEFINIC COMPOUNDS**
PHOTOCHLORIERUNGS- UND DEHYDROHALOGENIERUNGSVERFAHREN ZUR HERSTELLUNG VON OLEFINISCHEN VERBINDUNGEN
PROCEDE DE PHOTOCHLORURATION ET DE DESHYDROHALOGENATION POUR LA PREPARATION DE COMPOSES OLEFINIQUES

(30) Priority: 22.12.2004 US 638292 P
(43) Date of publication of application: 17.10.2007
(73) Proprietor: E.I. DU PONT DE NEMOURS AND COMPANY, Wilmington, DE 19898 (US)
(72) Inventor: RAO, Velliyur, Nott, Mallikarjuna, Wilmington, DE 19809 (US); SIEVERT, Allen, C., Elkton, MD 21921 (US)
(74) Representative: Matthews, Derek Peter
(86) International application number: PCT/US2005/046262
(87) International publication number: WO 2006/069103

(56) References cited:
- US-A- 3 330 881
- US-A- 4 855 112
- US-A- 5 763 706
- US-B1- 6 827 911

## Description

### FIELD OF THE INVENTION

This invention relates to the field of dehydrohalogenating chlorine-containing compounds, and particularly to materials suitable for use in producing the chlorine-containing compounds used in dehydrohalogenation by a photochlorination process.

### BACKGROUND OF THE INVENTION

Photochemical reactions use light as a source of energy to promote chemical processes. Ultraviolet (UV) and visible light are widely used in chemical synthesis both in laboratories and in commercial manufacturing. Well known photochemical reactions include photodimerization, photopolymerization, photohalogenation, photoisomerization and photodegradation. For example, cyclobutanetetracarboxylic dianhydride can be synthesized by photodimerization of maleic anhydride in a glass reactor using a mercury UV lamp (P. Boule et al., Tetrahedron Letters, Volume 11, pages 865 to 868, (1976)). Most of the vitamin D production in the United States is based on UV photolysis in a quartz vessel using light between 275 and 300 nm.

In photochlorination, chlorine (Cl₂) reacts with a saturated or unsaturated starting material, in the presence of a ultraviolet light source. This process is widely used to form carbon-chlorine bonds under mild conditions (e.g., room temperature) compared to the elevated temperatures normally required for thermal chlorination (R. Roberts et al., Applications of Photochemistry, TECHNOMIC Publishing Co., Inc. 1984.). For example, E. Tschuikow-Roux, et al. (J. Phys. Chem., Volume 88, pages 1408 to 1414 (1984)) report photochlorination of chloroethane and Walling et al.(J. Amer. Chem. Soc., Volume 79, pages 4181 to4187 (1957)) report photochlorination of certain substituted toluenes. U.S. Patent No. 5,190,626 describes the use of photochlorination in removing unsaturated compounds such as vinylidine chloride from CCl₂FCH₃ product. Chlorine-containing compounds such as CCl₂FCH₃ may be readily converted to olefinic compounds (e.g., CClF=CH₂) by dehydrohalogenation.

Typically in photochlorinations, light from a suitable source (e.g., an incandescent bulb or a UV lamp) is directed through a reactor wall to interact with the reactants therein. The portion of the reactor wall through which the light passes must have a suitable transmittance to allow light of a wavelength required for the photochlorination to enter the reactor. Typically, quartz or borosilicate glass like Pyrex^{™} glass have been employed as transparent materials. Quartz is expensive, but has a low cut-off wavelength at about 160 nm; Pyrex^{™} glass is less expensive, but has a relatively high cut-off wavelength at about 275 nm. Due to their reactivity, quartz and Pyrex are not appropriate materials of construction for chemical reactions involving base or HF. There is a need for additional materials which can be used for this purpose in photochemical reactions (e.g., photochlorinations).

U.S. Patent No. 3 330 881 discloses a two stage reaction in which hydrocarbons are successively halogenated and dehydrohalogenated. However, no details of the halogenation stage are given.

U.S. Patents Nos. 4 855 112 and 5 763 706 disclose reactors of highly resistant materials lined with fluoropolymer.

### SUMMARY OF THE INVENTION

This invention provides a process for producing an olefinic compound from at least one compound selected from hydrocarbons and halohydrocarbons containing at least two carbon atoms and at least two hydrogen atoms, comprising:
(a) directing light from a light source through the wall of a reactor to interact with reactants comprising chlorine and said at least one compound in said reactor, thereby producing a saturated halogenated hydrocarbon having increased chlorine content by photochlorination; and
(b) dehydrohalogenating said saturated halogenated hydrocarbon produced by the photochlorination in (a);
wherein the reactor is a tubular reactor or tank consisting of a poly(perhalo-olefin) polymer or a tube or tank fabricated from an opaque material with a window consisting of a poly(perhaloolefin) polymer, and the light directed through the reactor wall is directed through said poly(perhaloolefin) polymer.

### DETAILED DESCRIPTION

In accordance with this invention poly(perhaloolefin) polymers are used as photochlorination reactor materials through which light is able to pass for the purpose of interacting with the reactants, thereby promoting the photochlorination reaction. Preferred poly(perhaloolefin) polymers include perfluorinated polymers. Of note are embodiments where the poly(perhaloolefin) polymer is PTFE (i.e., poly(tetrafluoroethylene)). Also of note are embodiments where the poly(perhaloolefin)polymer is FEP (i.e., a copolymer of tetrafluoroethylene with hexafluoropropylene).

Perfluoropolymers have excellent chemical resistance, low surface energy, low flammability, low moisture adsorption, excellent weatherability and high continuous use temperature. In addition, they are among the purest polymer materials and are widely used in the semiconductor industry. They are also excellent for UV-vis transmission. For instance, a film of PFA copolymer (copolymers of tetrafluoroethylene and perfluoroalkyl vinyl ether) having a thickness of 0.025 mm has transmission of 91-96% for visible light between 400 to 700 nm and transmission of 77-91 % for UV light between 250 to 400 nm. Transmission of visible light through FEP is similar to PFA and UV light transmission of FEP is slightly better than PFA.

A suitable photochlorination apparatus includes a reactor in which light having a suitable wavelength (e.g., from about 250 nm to about 400 nm) can irradiate the reaction components for a time sufficient to convert at least a portion of the starting materials to one or more compounds having a higher chlorine content. The reactor may be, for example, a tubular reactor fabricated from poly(perhaloolefin) polymer (e.g., either a coil or extended tube), or tank fabricated from poly(perhaloolefin) polymer, or a tube or tank fabricated from an opaque material which has a window fabricated from poly(perhaloolefin) polymer. Typically, the thickness of the poly(perhaloolefin) polymer is sufficient to permit transmittance of the light of sufficient intensity to promote the reaction (e.g., 0.02 mm to 1 mm).

The apparatus also includes a light source. The light source may be any one of a number of arc or filament lamps known in the art. The light source is situated such that light having the desired wavelength may introduced into the reaction zone (e.g., a reactor wall or window fabricated from a poly(perhaloolefin) polymer and suitably transparent to light having a wavelength of from about 250 nm to about 400 nm).

Ordinarily the apparatus also includes a chlorine (Cl₂) source and a source of the material to be chlorinated. The chlorine source may be, for example, a cylinder containing chlorine gas or liquid, or equipment that produces chlorine (e.g., an electrochemical cell) that is connected to the reactor. The source of the material to be chlorinated may be, for example, a cylinder or pump fed from a tank containing the material, or a chemical process that produces the material to be chlorinated.

### Increasing Chlorine Content

In step (a) of the process of this invention the chlorine content of a halogenated hydrocarbon compound or a hydrocarbon compound is increased by reacting said compound with chlorine (Cl₂) in the presence of light.

Halogenated hydrocarbon compounds suitable as starting materials for the chlorination process of this invention may be saturated or unsaturated. Saturated halogenated hydrocarbon compounds suitable for the chlorination processes of this invention include those of the general formula CₙHₐBr_{b}Cl_{c}F_{d}, wherein n is an integer from 1 to 4, a is an integer from 1 to 9, b is an integer from 0 to 4, c is an integer from 0 to 9, d is an integer from 0 to 9, the sum of b, c and d is at least 1 and the sum of a, b, c, and d is equal to 2n + 2. Saturated hydrocarbon compounds suitable for chlorination are those which have the formula C_{q}Hᵣ where q is an integer from 1 to 4 and r is 2q + 2. Unsaturated halogenated hydrocarbon compounds suitable for the chlorination processes of this invention include those of the general formula CₚHₑBr_{f}Cl_{g}Fₕ, wherein p is an integer from 2 to 4, e is an integer from 0 to 7, f is an integer from 0 to 2, g is an integer from 0 to 8, h is an integer from 0 to 8, the sum of f, g and h is at least 1 and the sum of e, f, g, and h is equal to 2p. Unsaturated hydrocarbon compounds suitable for chlorination are those which have the formula CᵢHⱼ where i is an integer from 2 to 4 and j is 2i. The chlorine content of saturated compounds of the formula CₙHₐBr_{b}Cl_{c}F_{d} and C_{q}Hᵣ and/or unsaturated compounds of the formula CₚHₑBr_{f}Cl_{g}Fₕ and CᵢHⱼ may be increased by reacting said compounds with Cl₂ in the vapor phase in the presence of light. Such a process is referred to herein as a photochlorination reaction.

The photochlorination of the present invention may be carried out in either the liquid or the vapor phase. For vapor phase photochlorination, initial contact of the starting materials with Cl₂ may be a continuous process in which one or more starting materials are vaporized (optionally in the presence of an inert carrier gas, such as nitrogen, argon, or helium) and contacted with chlorine vapor in a reaction zone. A suitable photochlorination reaction zone is one in which light having a wavelength of from about 300 nm to about 400 nm can irradiate the reaction components for a time sufficient to convert at least a portion of the starting materials to one or more compounds having a higher chlorine content. The source of light may be any one of a number of arc or filament lamps known in the art. Light having the desired wavelength may introduced into the reaction zone by a number of means. For example, the light may enter the reaction zone through a lamp well or window fabricated from a poly(perhaloolefin) polymer suitably transparent to light having a wavelength of from about 300 nm to about 400 nm. Likewise, the walls of the reaction zone may be fabricated from such a material so that at least a portion of the light used for the photochlorination can be transmitted through the walls.

Alternatively, the process of the invention may be carried out in the liquid phase by feeding Cl₂ to a reactor containing the starting materials. Suitable liquid phase reactors include vessels fabricated from a poly(perhaloolefin) polymer in which an external lamp is directed toward the reactor and metal, glass-lined metal or fluoropolymer-lined metal reactors having one or more weiis or windows fabricated from a poly(perhaloolefin) polymer for introducing light having a suitable wavelength. Preferably the reactor is provided with a condenser or other means of keeping the starting materials in the liquid state while permitting the hydrogen chloride (HCl) released during the chlorination to escape the reactor.

In some embodiments it may be advantageous to conduct the photochlorination in the presence of a solvent capable dissolving one or more of the starting materials and/or chlorination products. Preferred solvents include those that do not have easily replaceable hydrogen substituents. Examples of solvents suitable for step (a) include carbon tetrachloride, 1,1-dichlorotetrafluoroethane, 1,2-dichlorotetrafluoroethane, 1,1,2-trichlorotrifluoroethane, benzene, chlorobenzene, dichlorobenzene, fluorobenzene, and difluorobenzene.

Suitable temperatures for the photochlorination of the starting materials of the formula are typically within the range of from about -20°C to about 60°C. Preferred temperatures are typically within the range of from about 0°C to about 40°C. In the liquid phase embodiment, it is convenient to control the reaction temperature so that starting material is primarily in the liquid phase; that is, at a temperature that is below the boiling point of the starting material(s) and product(s).

The pressure in a liquid phase process is not critical so long as the liquid phase is maintained. Unless controlled by means of a suitable pressure-regulating device, the pressure of the system increases as hydrogen chloride is formed by replacement of hydrogen substituents in the starting material by chlorine substituents. In a continuous or semi-batch process it is possible to set the pressure of the reactor in such a way that the HCl produced in the reaction is vented from the reactor (optionally through a packed column or condenser). Typical reactor pressures are from about 14.7 psig (101.3 kPa) to about 50 psig (344.6 kPa).

The amount of chlorine (Cl₂) fed to the reactor is based on whether the starting material(s) to be chlorinated is(are) saturated or unsaturated, and the number of hydrogens in CₙHₐBr_{b}Cl_{c}F_{d}, C_{q}Hᵣ, CₚHₑBr_{f}Cl_{g}Fₕ, and CᵢHⱼ that are to be replaced by chlorine. One mole of Cl₂ is required to saturate a carbon-carbon double bond and a mole of Cl₂ is required for every hydrogen to be replaced by chlorine. A slight excess of chlorine over the stoichiometric amount may be necessary for practical reasons, but large excesses of chlorine will result in complete chlorination of the products. The ratio of Cl₂ to halogenated carbon compound is typically from about 1:1 to about 10:1.

Specific examples of photochlorination reactions of saturated halogenated hydrocarbon compounds of the general formula CₙHₐBr_{b}Cl_{c}F_{d} and saturated hydrocarbon compounds of the general formula C_{q}Hᵣ which may be carried out in accordance with this invention include the conversion of C₂H₆ to a mixture containing CH₂ClCCl₃, the conversion of CH₂ClCF₃ to a mixture containing CHCl₂CF₃, the conversion of CCl₃CH₂CH₂Cl, CCl₃CH₂CHCl₂, CCl₃CHClCH₂Cl or CHCl₂CCl₂CH₂Cl to a mixture containing CCl₃CCl₂CCl₃, the conversion of CH₂FCF₃ to a mixture containing CHClFCF₃ and CCl₂FCF₃, the conversion of CH₃CHF₂ to CCl₃CClF₂, the conversion of CF₃CHFCHF₂ to a mixture containing CF₃CClFCHF₂ and CF₃CHFCClF₂, and the conversion of CF₃CH₂CHF₂ to CF₃CH₂CClF₂.

Specific examples of photochlorination reactions of unsaturated halogenated hydrocarbon compounds of the general formula CₚHₑBr_{f}Cl_{g}Fₕ and unsaturated hydrocarbon compounds of the general formula CᵢHⱼ which may be carried out in accordance with this invention include the conversion of C₂H₄ to a mixture containing CH₂ClCH₂Cl, the conversion of C₂Cl₄ to a mixture containing CCl₃CCl₃, the conversion of C₃H₆ a mixture containing CCl₃CCl₂CCl₃, and the conversion of CF₃CCl=CCl₂ to a mixture containing CF₃CCl₂CCl₃.

Of note is a photochlorination process for producing a mixture containing 2-chloro-1,1,1-trifluoroethane (i.e., CH₂ClCF₃ or HCFC-133a) by reaction of CH₃CF₃ with Cl₂ in the vapor phase in the presence of light in accordance with this invention. Also of note is a catalytic process for producing a mixture containing 1,2,2-trichloro-1,1,3,3,3-pentafluoropropane (i.e., CClF₂CCl₂CF₃ or CFC-215aa) or 1,2-dichloro-1,1,1,3,3,3-hexafluoropropane (i.e., CClF₂CClFCF₃ or CFC-216ba) by the chlorination of a corresponding hexahalopropene of the formula C₃Cl₆₋ₓFₓ, wherein x equals 5 or 6.

Contact times of from 0.1 to 60 seconds are typical; and contact times of from 1 to 30 seconds are often preferred.

Mixtures of saturated hydrocarbon compounds and saturated halogenated hydrocarbon compounds and mixtures of unsaturated hydrocarbon compounds and unsaturated halogenated hydrocarbon compounds as well as mixtures comprising both saturated and unsaturated compounds may be chlorinated in accordance with the present invention. Specific examples of mixtures of saturated and unsaturated hydrocarbons and halogenated hydrocarbons that may be used include a mixture of CCl₂=CCl₂ and CCl₂=CClCCl₃, a mixture of CHCl₂CCl₂CH₂Cl and CCl₃CHClCH₂Cl, a mixture of CHCl₂CH₂CCl₃ and CCl₃CHClCH₂Cl, a mixture of CHCl₂CHClCCl₃, CCl₃CH₂CCl₃, and CCl₃CCl₂CH₂Cl, a mixture of CHF₂CH₂CF₃ and CHCl=CHCF₃, and a mixture of CH₂=CH₂ and CH₂=CHCH₃.

### Producing Olefins

In step (b) of the process of this invention the saturated halogenated hydrocarbon produced in step (a) is dehydrohalogenated. Dehydrohalogenation reactions are well known in the art. They can be conducted both in either the vapor phase or liquid phase using a variety of catalysts. See for example, Milos Hudlicky, Chemistry of Organic Fluorine Compounds 2nd (Revised Edition), pages 489 to 495 and references cited therein (Ellis Harwood-Prentice Hall Publishers, 1992). Of note are vapor phase dehydrohalogenations in the presence of a catalyst. Suitable catalysts for dehydrohalogenation include carbon, metals (including elemental metals, metal oxides, metal halides, and/or other metal salts); alumina; fluorided alumina; aluminum fluoride; aluminum chlorofluoride; metals supported on alumina; metals supported on aluminum fluoride or chlorofluoride; magnesium fluoride supported on aluminum fluoride; metals supported on fluorided alumina; alumina supported on carbon; aluminum fluoride or chlorofluoride supported on carbon; fluorided alumina supported on carbon; metals supported on carbon; and mixtures of metals, aluminum fluoride or chlorofluoride, and graphite. Suitable metals for use on catalysts (optionally on alumina, aluminum fluoride, aluminum chlorofluoride, fluorided alumina, or carbon) include chromium, iron, and lanthanum. Preferably when used on a support, the total metal content of the catalyst will be from about 0.1 to 20 percent by weight; typically from about 0.1 to 10 percent by weight. Preferred catalysts for dehydrohalogenation include carbon, alumina, and fluorided alumina.

Halogenated hydrocarbon compounds suitable for the dehydrohalogenation of this invention include saturated compounds of the general formula CₘH_{w}BrₓCl_{y}F_{z}, wherein m is an integer from 2 to 4, w is an integer from 1 to 9, x is an integer from 0 to 4, y is an integer from 1 to 9, z is an integer from 0 to 8, and the sum of w, x, y, and z is equal to 2n + 2. The compound photochlorinated to produce the compound subjected to dehydrohalogenation (e.g., a saturated compound of the formula CₙHₐBr_{b}Cl_{c}F_{d} or a saturated compound of the formula C_{q}Hᵣ as described above) should contain at least two carbon atoms and two hydrogen atoms (e.g., for said compounds of the formulas CₙHₐBr_{b}Cl_{c}F_{d} and C_{q}Hᵣ w, n, a and q should be at least 2). Of note are processes where the compound photochlorinated is a halogenated hydrocarbon that contains fluorine

Of note is a process for producing 1,1-difluoroethylene (i.e., CF₂=CH₂ or vinylidene fluoride) by the photochlorination of 1,1-difluoroethane (i.e., CHF₂CH₃ or HFC-152a) to produce 1-chloro-1,1-difluoroethane (i.e., CClF₂CH₃ or HCFC-142b); and the dehydrohalogenation of the HCFC-142b to produce 1,1-difluoroethylene. Also of note is a process for producing tetrafluoroethylene (i.e., CF₂=CF₂) by the photochlorination of 1,1,2,2-tetrafluoroethane (i.e., CHF₂CHF₂ or HFC-134) to produce 2-chloro-1,1,2,2-tetrafluoroethane (i.e., CClF₂CHF₂ or HCFC-124a); and the dehydrohalogenation of the HCFC-124a to produce tetrafluoroethylene. Also of note is a process for producing hexafluoropropylene (CF₃CF=CF₂) by the photochlorination of 1,2-dihydrohexafluoropropane (i.e., CF₃CHFCHF₂ or HFC-236ea) to produce 1-chloro-1,1,2,3,3,3-hexafluoropropane (i.e., CF₃CHFCClF₂ or HCFC-226ea); and dehydrohalogenation of the HCFC-226ea to produce hexafluoropropylene.

### EXAMPLES

General Procedure for Chlorination and Product Analysis Photochlorination was carried out using a 110 volt/275 watt sunlamp placed (unless otherwise specified) at a distance of 0.5 inches (1.3 cm) from the outside of the first turn of the inlet end of a coil of fluoropolymer tubing material through which the materials to be chlorinated were passed. Two fluoropolymer tubes were used in the examples below. One tube was fabricated from PTFE (18 inches(45.7 cm) long X 1/16" (16 mm) OD X 0.038" (0.97 mm) ID) which was coiled to a diameter of 2.5 inches (6.4 cm) and contained suitable feed and exit ports. The other tube was fabricated from FEP (18 inches (45.7 cm) X 0.125" (3.2 mm) OD X 0.085" (2.2 mm) ID) which was coiled to a diameter of 3 inches (7.6 cm) and contained suitable feed and exit ports. The organic feed material and chlorine were fed to the tubing using standard flow-measuring devices. The gas mixture inside was exposed to light generated by the sunlamp. The experiments were conducted at ambient temperature (about 23°C) and under about atmospheric pressure. Organic feed material entering the tubing and the product after photochlorination were analyzed on-line using a GC/MS. The results are reported in mole%.

PTFE (poly(tetrafluoroethylene) is a linear homopolymer of tetrafluoroethylene (TFE). FEP is a copolymer of tetrafluoroethylene and hexafluoropropylene. CFC-114 is CClF₂CClF₂. CFC-114a is CF3CCl₂F. HCFC-132b is CClF₂CH₂Cl. HCFC-142 is CHF₂CHCl₂. CFC-216ba is CF₃CClFCCF₂. HCFC-226ba is CF₃CClFCHF₂. HCFC-235fa is CF₃CH₂CClF₂. HFC-245fa is CF₃CH₂CHF₂.

### Example 1

### Photochlorination of HFC-134a

Feed gases consisting of HFC-134a at a flow rate of 5.0 sccm (8.3(10)⁻⁸ m³/sec) and chlorine gas at a flow rate of 2.5 sccm (4.2(10)⁻⁸ m³/sec) were introduced into the PTFE tubing. After exposure to light for one hour, the product was analyzed and found to contain 68.1 mole % of HFC 134a, 24.2 mole % of HCFC-124, 7.0 mole % of CFC-114a and 0.7 mole % of other unidentified compounds. The molar yield of CFC-114a compared to the total amount of CFC-114a and HCFC-124 was 22.4 %.

### Example 2

### Photochlorination of HFC-134a

Feed gases consisting of HFC-134a at a flow rate of 5.0 sccm (8.3(10)⁻⁸ m³/sec) and chlorine gas at a flow rate of 7.5 sccm (1.3(10)⁻⁷ m³/sec) were introduced into the PTFE tubing. After exposure to light for one hour, the product was analyzed and found to contain 61.4 mole % of HFC-134a, 27.5 mole % of HCFC-124, 10.7 mole % of CFC -114a and 0.4 mole % of other unidentified compounds. The molar yield of CFC-114a compared to the total amount of CFC-114a and HCFC-124 was 28.0 %.

### Example 3

### Photochlorination of HFC-134a

Feed gases consisting of HFC-134a at a flow rate of 5.0 sccm (8.3(10)⁻⁸ m³/sec) and chlorine gas at a flow rate of 2.5 sccm (4.2(10)⁻⁸ m³/sec) were introduced into the FEP tubing. After exposure to light for one hour, the product was analyzed and found to contain 53.0 mole % of HFC-134a, 30.0 mole % of HCFC-124, 16.2 mole % of CFC-114a and 0.8 mole % of other unidentified compounds. The molar yield of CFC-114a compared to the total amount of GFC-114a and HCFC-124 was 35.0 %.

### Example 4

### Photochlorination of HFC-134a

In this experiment, the distance of the lamp from the coiled tube was 1.5 inches (3.8 cm). Feed gases consisting of HFC-134a at a flow rate of 5.0 sccm (8.3(10)⁻⁸ m³/sec) and chlorine gas at a flow rate of 2.5 sccm (4.2(10)⁻⁸ m³/sec) were introduced into the FEP tubing. After exposure to light for one hour, the product was analyzed and found to contain 56.5 mole % of HFC-134a, 29.0 mole % of HCFC-124, 14.0 mole % of HCFC 114a and 0.5 mole % of other unidentified compounds. The molar yield of HCFC 114a compared to the total amount of HCFC 114a and HCFC 124 was 32.6 %.

### Example 5

### Photochlorination of HFC 134a

In this experiment, the distance of the lamp from the coiled tube was 3.0 inches (7.6 cm). Feed gases consisting of HFC-134a at a flow rate of 5.0 sccm (8.3(10)⁻⁸ m³/sec) and chlorine gas at a flow rate of 2.5 sccm (4.2(10)⁻⁸ m³/sec) were introduced into the FEP tubing. After exposure to light for one hour, the product was analyzed and found to contain 63.8 mole % of HFC-134a, 26.0 mole % of HCFC-124, 9.5 mole % of CFC-114a and 0.7 mole % of other unidentified compounds. The molar yield of CFC-114a compared to the total amount of CFC-114a and HCFC-124 was 26.8 %.

### Example 6

### Photochlorination of HFC-152a

In this experiment, the distance of the lamp from the coiled tube was 0.5 inch (1.3 cm). Feed gases consisting of HFC-152a at a flow rate of 5.0 sccm (8.3(10)⁻⁸ m³/sec) and chlorine gas at a flow rate of 2.5 sccm (4.2(10)⁻⁸ m³/sec) were introduced into the FEP tubing. After exposure to light for one hour, the product was analyzed and found to contain 30.5 mole % of HFC-152a, 67.7 mole % of HCFC-142b, 1.0 mole % of HCFC-142, 0.2 mole % of HCFC-132b and 0.6 mole % of other unidentified compounds.

### Example 7

### Photochlorination of HFC-152a

In this experiment, the distance of the lamp from the coiled tube was 3.0 inches (7.6 cm). Feed gases consisting of HFC-152a at a flow rate of 5.0 sccm (8.3(10)⁻⁸ m³/sec) and chlorine gas at a flow rate of 2.5 sccm (4.2(10)⁻⁸ m³/sec) were introduced into the FEP tubing. After exposure to light for one hour, the product was analyzed and found to contain 27.9 mole % of HFC-152a, 70.1 mole % of HCFC-142b, 0.9 mole % of HCFC-142, 0.2 mole % of HCFC- 132b and 0.9 mole % of other unidentified compounds.

### Example 8

### Photochlorination of HFC-134

Feed gases consisting of HFC-134 at a flow rate of 5.0 sccm (8.3(10)⁻⁸ m³/sec) and chlorine gas at a flow rate of 2.5 sccm (4.2(10)⁻⁸ m³/sec) were introduced into the PTFE tubing. After exposure to light for one hour, the product was analyzed and found to contain 43.4 mole % of HFC 134, 50.8 mole % of HCFC-124a, 5.3 mole % of CFC-114 and 0.5 mole % of other unidentified compounds. The molar yield of CFC-114 compared to the total amount of CFC-114 and HCFC-124a was 9.4 %.

### Example 9

### Photochlorination of HFC 236ea

Feed gases consisting of HFC-236ea at a flow rate of 5.0 sccm (8.3(10)⁻⁸ m³/sec) and chlorine gas at a flow rate of 2.5 sccm (4.2(10)⁻⁸ m³/sec) were introduced into the PTFE tubing. After exposure to light for one hour, the product was analyzed and found to contain 59.7 mole % of HFC-236ea, 5.6 mole % of HCFC-226ba, 33.6 mole % of HCFC-226ea, 0.7 mole % of CFC-216ba and 0.4 mole % of other unidentified compounds.

### Example 10

### Photochlorination of HFC 236ea

Feed gases consisting of HFC-236ea at a flow rate of 5.0 sccm (8.3(10)⁻⁸ m³/sec) and chlorine gas at a flow rate of 7.5 sccm (1.3(10)⁻⁷ m³/sec) were introduces into the PTFE tubing. After exposure to light for one hour, the product was analyzed and found to contain 61.4 mole % of HFC-236ea, 5.5 mole % of HCFC-226ba, 31.9 mole % of HCFC-226ea, 0.7 mole % of CFC-216ba and 0.5 mole % of other unidentified compounds.

### Example 11

### Photochlorination of HFC-245fa

HFC-245fa was analyzed prior to chlorination to have a purity of 99.8 %. Feed gases consisting of HFC-245fa at a flow rate of 3.5 sccm (5.8(10)⁻⁸ m³/sec) and chlorine gas at a flow rate of 3.5 sccm (5.8(10)⁻⁸ m³/sec) were introduced into the PTFE tubing. After exposure to light for one hour, the product was analyzed and found to contain 65.8 mole % of HFC-245fa, 33.0 mole % of HCFC-235fa, and 1.2 mole % of other unidentified compounds.

### Example 12

### Photochlorination of HFC-245fa

HFC-245fa was analyzed prior to chlorination to have a purity of 99.8 %. Feed gases consisting of HFC-245fa at a flow rate of 5.0 sccm (8.3(10)⁻⁸ m³/sec) and chlorine gas at a flow rate of 2.5 sccm (4.2(10)⁻⁸ m³/sec) were introduced into the FEP tubing. After exposure to light for one hour, the product was analyzed and found to contain 18.6 mole % of HFC-245fa, 81.0 mole % of HCFC-235fa, and 0.4 mole % of other unidentified compounds.

## Claims

1. A process for producing an olefinic compound from at least one compound selected from hydrocarbons and halohydrocarbons containing at least two carbon atoms and at least two hydrogen atoms, comprising:
(a) directing light from a light source through the wall of a reactor to interact with reactants comprising chlorine and said at least one compound in said reactor, thereby producing a saturated halogenated hydrocarbon having increased chlorine content by photochlorination; and
(b) dehydrohalogenating said saturated halogenated hydrocarbon produced by the photochlorination in (a);
wherein the reactor is a tubular reactor or tank consisting of a poly(perhaloolefin) polymer or a tube or tank fabricated from an opaque material with a window consisting of a poly(perhaloolefin) polymer, and the light directed through the reactor wall is directed through said poly(perhaloolefin) polymer.

2. The process of Claim 1 wherein the poly(perhaloolefin) polymer is a perfluorinated polymer.

3. The process of Claim 2 wherein the poly(perhaloolefin) polymer is poly(tetrafluoroethylene).

4. The process of Claim 2 wherein the poly(perhaloolefin) polymer is a copolymer of tetrafluoroethylene and hexafluoropropylene.

5. The process of Claim 1 wherein in (a) a compound containing fluorine is photochlorinated.

6. The process of Claim 5 wherein in (a) CH₃CHF₂ is photochlorinated to produce CH₃CClF₂; and wherein in (b) CH₃CClF₂ is dehydrohalogenated to produce CF₂=CH₂.

7. The process of Claim 5 wherein in (a) CHF₂CHF₂ is photochlorinated to produce CHF₂CClF₂; and wherein in (b) CHF₂CClF₂ is dehydrohalogenated to produce CF₂=CF₂.

8. The process of Claim 5 wherein in (a) CF₃CHFCHF₂ is photochlorinated to produce CF₃CHFCClF₂; and wherein in (b) CF₃CHFCClF₂ is dehydrohalogenated to produce CF₃CF=CF₂

9. The process of any one of Claims 1 to 8 wherein step (a) is carried out in the liquid phase.

10. The process of Claim 9 wherein the reaction is controlled to keep the starting materials of step (a) in the liquid phase while permitting hydrogen chloride to escape.

11. The process of Claim 9 or Claim 10 wherein the step (a) is conducted in the presence of a solvent capable of dissolving one or more of the starting materials and/or chlorination products.

12. The process of any one of Claims 1 to 8 wherein step (a) is carried out in the vapor phase.

13. The process of Claim 12 wherein step (a) is carried out in the presence of an inert carrier gas.

14. The process of any one of Claims 1 to 5 and 9 to 13 wherein a saturated halogenated hydrocarbon of the formula CₙHₐBr_{b}Cl_{c}F_{d}, wherein n is an integer from 1 to 4, a is an integer from 1 to 9, b is an integer from 0 to 4, c is an integer from 0 to 9, d is an integer from 0 to 9, the sum of b, c and d is at least 1 and the sum of a, b, c, and d is equal to 2n + 2, is photochlorinated in step (a).

15. The process of Claim 14 wherein CH₂ClCF₃ is converted to a mixture containing CHCl₂CF₃; CCl₃CH₂CH₂Cl, CCl₃CH₂CHCl₂, CCl₃CHClCH₂Cl or CHCl₂CCl₂CH₂Cl is converted to a mixture containing CCl₃CCl₂CCl₃; CH₂FCF₃ is converted to a mixture containing CHClFCF₃ and CCl₂FCF₃; CH₃CHF₂ is converted to CCl₃CClF₂; CF₃CHFCHF₂ is converted to a mixture containing CF₃CClFCHF₂ and CF₃CHFCClF₂; or CF₃CH₂CHF₂ is converted to CF₃CH₂CCIF₂.

## Patentansprüche

1. Verfahren zur Herstellung einer olefinischen Verbindung aus mindestens einer Verbindung ausgewählt unter Kohlenwasserstoffen und Halogenkohlenwasserstoffen, die mindestens zwei Kohlenstoffatome und mindestens zwei Wasserstoffatome enthalten, umfassend:
(a) das Leiten von Licht aus einer Lichtquelle durch die Wand eines Reaktors, um mit Reaktanden, die Chlor und die mindestens eine Verbindung umfassen, in dem Reaktor in Wechselwirkung zu treten, wodurch ein gesättigter halogenierter Kohlenwasserstoff, der einen erhöhten Chlorgehalt aufweist, durch Photochlorierung hergestellt wird; und
(b) das Dehydrohalogenieren des durch die Photochlorierung unter (a) hergestellten gesättigten halogenierten Kohlenwasserstoffs;
wobei der Reaktor ein Röhrenreaktor oder -tank ist, bestehend aus einem Poly(perhaloolefin)polymer oder eine Röhre oder ein Tank ist, der/die aus einem opaken Material, mit einem Fenster bestehend aus einem Poly(perhaloolefin)polymer hergestellt ist, und das durch die Reaktorwand geleitete Licht durch das Poly(perhaloolefin)polymer geführt wird.

2. Verfahren nach Anspruch 1, wobei das Poly(perhaloolefin)polymer ein perfluoriertes Polymer ist.

3. Verfahren nach Anspruch 2, wobei das Poly(perhaloolefin)polymer Poly(tetrafluorethylen) ist.

4. Verfahren nach Anspruch 2, wobei das Poly(perhaloolefin)polymer ein Copolymer von Tetrafluorethylen und Hexafluorpropylen ist.

5. Verfahren nach Anspruch 1, wobei in (a) eine Verbindung, die Fluor enthält, photochloriert wird.

6. Verfahren nach Anspruch 5, wobei in (a) CH₃CHF₂ zur Bildung von CH₃CClF₂ photochloriert wird; und wobei in (b) CH₃CClF₂ zur Bildung von CF₂=CH₂ dehydrohalogeniert wird.

7. Verfahren nach Anspruch 5, wobei in (a) CHF₂CHF₂ zur Bildung von CHF₂CClF₂ photochloriert wird; und wobei in (b) CHF₂CClF₂ zur Bildung von CF₂=CF₂ dehydrohalogeniert wird.

8. Verfahren nach Anspruch 5, wobei in (a) CF₃CHFCHF₂ zur Bildung von CF₃CHFCClF₂ photochloriert wird; und wobei in (b) CF₃CHFCClF₂ zur Bildung von CF₃CF=CF₂ dehydrohalogeniert wird.

9. Verfahren nach einem der Ansprüche 1 bis 8, wobei der Schritt (a) in der flüssigen Phase durchgeführt wird.

10. Verfahren nach Anspruch 9, wobei die Reaktion gesteuert wird, um die Ausgangsmaterialien von Schritt (a) in der flüssigen Phase zu halten, während man das Wasserstoffchlorid entweichen lässt.

11. Verfahren nach Anspruch 9 oder Anspruch 10, wobei der Schritt (a) in Gegenwart eines Lösungsmittels durchgeführt wird, das in der Lage ist, ein oder mehrere der Ausgangsmaterialien und/oder Chlorierungsprodukte zu lösen.

12. Verfahren nach einem der Ansprüche 1 bis 8, wobei der Schritt (a) in der Dampfphase durchgeführt wird.

13. Verfahren nach Anspruch 12, wobei der Schritt (a) in Gegenwart eines inerten Trägergases durchgeführt wird.

14. Verfahren nach einem der Ansprüche 1 bis 5 und 9 bis 13, wobei ein gesättigter halogenierter Kohlenwasserstoff der Formel CₙHₐBr_{b}Cl_{c}F_{d}, wobei n eine ganze Zahl von 1 bis 4, a eine ganze Zahl von 1 bis 9, b eine ganze Zahl von 0 bis 4, c eine ganze Zahl von 0 bis 9, d eine ganze Zahl von 0 bis 9, die Summe von b, c und d mindestens 1 und die Summe von a, b, c und d gleich 2n + 2 ist, in Schritt (a) photochloriert wird.

15. Verfahren nach Anspruch 14, wobei CH₂ClCF₃ zu einer Mischung umgewandelt wird, die CHCl₂CF₃ enthält; CCl₃CH₂CH₂Cl, CCl₃CH₂CHCl₂, CCl₃CHClCH₂Cl oder CHCl₂CCl₂CH₂Cl zu einer Mischung umgewandelt werden, die CCl₃CCl₂CCl₃ enthält; CH₂FCF₃ zu einer Mischung umgewandelt wird, die CHClFCF₃ und CCl₂FCF₃ enthält; CH₃CHF₂ zu CCl₃CClF₂ umgewandelt wird; CF₃CHFCHF₂ zu einer Mischung umgewandelt wird, die CF₃CClFCHF₂ und CF₃CHFCClF₂ enthält; oder CF₃CH₂CHF₂ zu CF₃CH₂CClF₂ umgewandelt wird.

## Revendications

1. Procédé de production d'un composé oléfinique à partir d'au moins un composé choisi parmi les hydrocarbures et les hydrocarbures halogénés contenant au moins deux atomes de carbone et au moins deux atomes d'hydrogène, comprenant :
(a) la direction de lumière à partir d'une source lumineuse à travers la paroi d'un réacteur pour interagir avec des réactifs comprenant du chlore et ledit au moins un composé dans ledit réacteur, produisant de là un hydrocarbure halogéné saturé ayant une teneur accrue en chlore par photochloration ; et
(b) la déshydrohalogénation dudit hydrocarbure halogéné saturé produit par la photochloration au point (a) ;
dans lequel le réacteur est un réacteur tubulaire ou une cuve constitué(e) d'un polymère poly(perhalooléfinique) ou un tube ou une cuve fabriqué(e) à partir d'un matériau opaque avec une fenêtre constituée d'un polymère poly(perhalooléfinique), et la lumière dirigée à travers la paroi du réacteur est dirigée à travers ledit polymère poly(perhalooléfinique).

2. Procédé selon la revendication 1, dans lequel le polymère poly(perhalooléfinique) est un polymère perfluoré.

3. Procédé selon la revendication 2, dans lequel le polymère poly(perhalooléfinique) est le poly(tétrafluoroéthylène).

4. Procédé selon la revendication 2, dans lequel le polymère poly(perhalooléfinique) est un copolymère de tétrafluoroéthylène et d'hexafluoropropylène.

5. Procédé selon la revendication 1, dans lequel en (a) un composé contenant du fluor est photochloré.

6. Procédé selon la revendication 5, dans lequel en (a) CH₃CHF₂ est photochloré pour produire CH₃CClF₂ ; et dans lequel en (b) CH₃CClF₂ est déshydrohalogéné pour produire CF₂=CH₂.

7. Procédé selon la revendication 5, dans lequel en (a) CHF₂CHF₂ est photochloré pour produire CHF₂CClF₂ ; et dans lequel en (b) CHF₂CClF₂ est déshydrohalogéné pour produire CF₂=CF₂.

8. Procédé selon la revendication 5, dans lequel en (a) CF₃CHFCHF₂ est photochloré pour produire CF₃CHFCClF₂ ; et dans lequel en (b) CF₃CHFCClF₂ est déshydrohalogéné pour produire CF₃CF=CF₂.

9. Procédé selon l'une quelconque des revendications 1 à 8, dans lequel l'étape (a) est conduite en phase liquide.

10. Procédé selon la revendication 9, dans lequel la réaction est contrôlée pour maintenir les matériaux de départ de l'étape (a) dans la phase liquide tout en permettant au chlorure d'hydrogène de s'échapper.

11. Procédé selon la revendication 9 ou la revendication 10, dans lequel l'étape (a) est conduite en la présence d'un solvant capable de dissoudre un ou plusieurs des matériaux de départ et/ou des produits de chloration.

12. Procédé selon l'une quelconque des revendications 1 à 8, dans lequel l'étape (a) est conduite en phase vapeur.

13. Procédé selon la revendication 12, dans lequel l'étape (a) est conduite en présence d'un gaz porteur inerte.

14. Procédé selon l'une quelconque des revendications 1 à 5 et 9 à 13, dans lequel un hydrocarbure halogéné saturé de formule CₙHₐBr_{b}Cl_{c}F_{d}, dans lequel n est un nombre entier prenant une valeur allant de 1 à 4, a est un nombre entier prenant une valeur allant de 1 à 9, b est un nombre entier prenant une valeur de 0 à 4, c est un nombre entier prenant une valeur allant de 0 à 9, d est un nombre entier prenant une valeur de 0 à 9, la somme de b, c et d est d'au moins 1 et la somme de a, b, c et d est égale à 2n+2, est photochloré dans l'étape (a).

15. Procédé selon la revendication 14, dans lequel CH₂ClCF₃ est converti en un mélange contenant CHCl₂CF₃, CCl₃CH₂CH₂Cl, CCl₃CH₂CHCl₂, CCl₃CHClCH₂Cl ou CHCl₂CCl₂CH₂Cl est converti en un mélange contenant CCl₃CCl₂CCl₃ ; CH₂FCF₃ est converti en un mélange contenant CHClFCF₃ et CClFCF₃ ; CH₃CHF₂ est converti en CCl₃CClF₂ ; CF₃CHFCHF₂ est converti en un mélange contenant CF₃CClFCHF₂ et CF₃CHFCClF₂ ; ou CF₃CH₂CHF₂ est converti en CF₃CH₂CClF₂.
